# EUROPÄISCHE PATENTANMELDUNG

(11) **EP 1 072 677 A1**
(43) Veröffentlichungstag der Anmeldung: **31.01.2001**
(21) Anmeldenummer: 00115082.0
(22) Anmeldetag: 27.07.2000
(51) Int. Cl.: C12N 15/01, C12N 5/20, C07K 16/28

(54) **Aus der Zellinie 2.4G2 abstammende Hybridomzellinie, die bevorzugt keine Maus-kappa-Ketten produziert**

(30) Priorität: 29.07.1999 DE 19935794; 20.09.1999 DE 19944994
(71) Anmelder: GSF-Forschungszentrum für Umwelt und Gesundheit GmbH, 85764 Neuherberg (DE)
(72) Erfinder: Kummer, Udo, Dr., 80538 München (DE)
(74) Vertreter: Reinhard - Skuhra - Weise & Partner

(57) **Zusammenfassung**

Erfindungsgemäß wird eine aus der Zellinie 2.4G2 generierte Zellinie beschrieben, die keine oder zumindest weniger als 0,1 % an Zellen enthält, die Mischantikörper produzieren, die in einem oder beiden Bindungsarmen Maus-kappa-Ketten tragen.

## Beschreibung

Die vorliegende Erfindung betrifft eine Zellinie, die bevorzugt keine Maus-kappa-Ketten produziert und aus der Zellinie 2.4G2 abgeleitet ist.

Die Non-Profit Einrichtung 'American Type Culture Collection' (ATCC) vertreibt unter der Registrierungsnummer HB-197 ein Hybridom (2.4G2; siehe beiliegenden Ausdruck), das 1979 von J. Unkeless (*J Unkeless*; 1979. *J Exp Med 150:580-596*) mit Hilfe der P3U1 Myelomlinie generiert wurde. Es produziert einen monoklonalen Antikörper, der gegen die Ligandenbindungsstelle der niedrig-affinen FcγRezeptoren (FcγRezeptor II und III) der Maus gerichtet ist und so deren Funktion blockiert.

Diese Myelomlinie ist noch zur Eigensynthese von Maus-κ-Ketten befähigt, so daß die Hybridomlinie zwei grundsätzlich unterschiedliche Antikörper-Populationen produziert: eine Population, die ausschließlich von den Genen der Eltern-B-Zelle (Ratten-Milzzelle) kodiert ist und eine weitere Population (Misch-Antikörper), in die entweder in einem oder beiden Bindungsarmen die irrelevante Leichtkette der Maus eingebaut ist. Beide Antikörper-Populationen entstehen durch die willkürliche, im cisternalen Kompartiment der Hybridomzelle stattfindende Kombination der verschiedenen Schwer- und Leichtketten, die von den entsprechenden Genen der beiden Elternzellen kodiert werden. Der Einbau auch nur einer irrelevanten Leichtkette der Maus in den Antikörper verändert dessen Antigen-Bindungsstellen so grundlegend, daß eine produktive Bindung mit dem Antigen nicht mehr stattfinden kann. Numerisch dominieren bei solchen Hybridomen die unerwünschten Misch-Antikörper, während die Antikörper, die keine Mausanteile aufweisen, nur ¼ der gesamten produzierten Antikörpermenge ausmachen und erst durch komplizierte Reinigungsverfahren dargestellt werden können.

Es ist eine Aufgabe der vorliegenden Erfindung, eine neue Hybridomzellinie bereitzustellen, die die Vorteile der ursprünglichen Hybridomzellinie 2.4G2 besitzt, gleichzeitig jedoch keine unerwünschten Misch-Antikörper mehr produziert, so daß die Nachteile einer aufwendigen Reinigung entfallen können.

Diese Aufgabe wird erfindungsgemäß durch eine Hybridomzellinie gelöst, die sich aus der Linie 2.4G2 (ATCC HB-197) ableitet und die keine oder zumindest weniger als 0,1% an Zellen enthält, die Misch-Antikörper produzieren, die in einem oder beiden Bindungsarmen Maus-kappa-Ketten tragen.

In einer bevorzugten Ausführungsform produziert die Zellinie weniger als 0,05% und noch bevorzugter weniger als 0,01% an Misch-Antikörpern. Am bevorzugtesten ist die Zellinie 2.4G2uk, die bei der Deutschen Sammlung für Mikroorganismen und Zellkulturen gemäß dem Budapester Vertrag unter der Eingangsnummer DSM ACC 2379 am 17. Dezember 1998 gemäß der beiliegenden Empfangsbestätigung und Lebensfähigkeitsbescheinigung hinterlegt wurde.

Diese Zellinie produziert keine Misch-Antikörper mehr.

Die erfindungsgemäß bereitgestellten Zellinien werden dadurch hergestellt, daß die Ausgangszellinie 2.4G2 mit mutagenen Agenzien behandelt wird. Beispiele hierfür sind chemische Mutagene oder eine Strahlenbehandlung bspw. mit radioaktiven Strahlen oder Röntgenstrahlen. Durch anschließende Selektion wird eine Zellinie generiert, die keine oder zumindest weniger als 0,1%, bevorzugt weniger als 0,05%, insbesondere bevorzugt weniger als 0,01% an Zellen oder keine Zellen enthält, die Misch-Antikörper produzieren, die in einem oder beiden Bindungsarmen Maus-kappa-Ketten tragen.

Die erfindungsgemäß bereitgestellt Zellinie wird insbesondere im experimentellen Bereich für in vitro- und in vivo-Versuche eingesetzt, bei denen die Funktion der niedrig-affinen Fcγ-Rezeptoren FcγRII oder FcγRIII blockiert werden sollen.

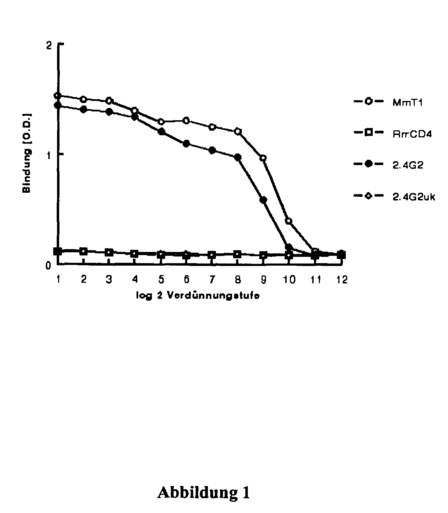

Nachfolgend wird die Erfindung noch detaillierter anhand eines Ausführungsbeispiels und einer Abbildung beschrieben. Die Abbildung 1 zeigt den Nachweis von Maus-kappa-Ketten in Antikörpern mit Hilfe eines direkten Immuntests.

Abbildung 1: Die log 2 Verdünnungen der Antikörper 2.4G2 und 2.4G2uk, sowie der Kontroll-Antikörper MmT1 (Antikörper mit Maus-κ-Kette) und RmCD4 (Antikörper ohne Maus-κ-Kette) wurden über Nacht an die Festphase gebunden. Dazu wurden die Antikörper jeweils auf 1,0 mg/ml mit dem Beschichtungspuffer (Phosphat-gepufferte Kochsalzlösung) verdünnt und ausgehend von der höchsten Antikörperkonzentration eine log 2 Verdünnungsreihe hergestellt. Jede Kavität wurde mit 100 µl Antikörperlösung beschickt. Nach mehreren Waschschritten werden die so vorbereiteten Kavitäten mit 100 µl eines Peroxidase-markiertem Antikörpers, der spezifisch gegen die Maus-κ-Kette gerichtet ist, inkubiert. Am Ende der Inkubationszeit (90 min) wurde die Platte wiederum gewaschen und den Kavitäten wurde 100 µl Substratlösung zugesetzt. Alle Inkubationen wurden bei Raumtemperatur ausgeführt.

Unerwarteterweise ist es erfindungsgemäß gelungen, aus dem Ursprungshybridom 2.4G2 einen Subklon zu isolieren, der keine Maus-Leichtketten mehr synthetisiert. Die von diesem Subklon sezernierten monoklonalen Antikörper gehören somit alle dem Typ an, der ausschließlich von den Genen der parentalen B-Zelle kodiert wird. Eine weitere Produktion der unerwünschten Misch-Antikörper findet nicht mehr statt.

Zur Isolierung des Subklons 2.4G2uk wurde wie folgt vorgegangen:

Genetische Defekte entstehen sporadisch durch Mutationen in permanenten Zelllinien. So liegt z.B. die Wahrscheinlichkeit für die spontane Entstehung von sog. Klassen-Switch-Varianten bei 10⁻⁵ bis 10⁻⁷ pro Generation (*HL Aguila*, *RR Pollock*, *G Spira*, *MD Scharf;*, *1986*. *Immunol Today 7:380-383*). Durch Zugabe von chemischen Mutagenen (Ethyl-Methansulfonat 100 µg/ml) oder durch ionisierende Strahlen (100-200 rad) kann die Mutationsrate erhöht werden (*G Hale*, *SP Cobbold*, *H Waldmann*, *G Easter*, *P Matejtschuk*, *RRA Coombs*; *1987*. *J Immunol Mehtods 103:59-67*). Anschließend selektiert man die Nachkommen hinsichtlich der gewünschten Eigenschaften und vermehrt sie.

Unser Vorgehen sah nun so aus, dass wir zunächst im Ursprungsklon 2.4G2 durch Bestrahlung mit 250 rad Mutationen im Erbgut erzeugt haben. Die so bestrahlten Hybridomzellen wurden auf mehrere mittlere Zellkulturflaschen (250 ml) überführt und für ca. 1 Monat in Kultur gehalten, wobei der jeweilige Zeitpunkt für das Umsetzen der Zellen bei einer Dichte von ca. 250 000 Zellen/ml erfolgte. Hinter diesem Vorgehen stand die Überlegung, die Nachkommenschaft der Strahlen-induzierten 2.4G2-Mutanten numerisch zu vergrößern. In Anschluß an das einmonatige Propagieren der Zellen wurde dann mit Hilfe der Limiting-Dilution-Klonierung versucht, aus dem strahlen-behandelten Ursprungshybridom die Zellen zu isolieren, die den gewünschten Antikörper produzieren. Zu diesem Zweck werden die den gewünschten Antikörper produzierenden Zellen der Primärkultur in die Kavitäten von frischen 96 well Zellkulturplatten ausgesät. Aufgrund einer Probeaussaat, die angibt, in welcher Dichte die Zellen ausgesät werden müssen, um die gewünschten Hybridome annähernd klonal anzuzüchten, wurden die Hybridomzellen in Medium so verdünnt, dass sich nach Aussaat - gemäss der Poisson-Verteilung - in jeder Kavität der 96 well Zellkulturplatte höchstens eine Zelle befand. Durch eine mikroskopische Kontrolle (Durchlichtmikroskop) konnte das Heranwachsen eines Klons (Monoklonalität) sichergestellt werden.

Zur Identifizierung des gewünschten 2.4G2-Antikörper wurden die Zellkulturüberstände der heranwachsenden Klone getestet. Der primäre Screening-Test, der kostengünstig ist und einen hohen Probendurchsatz gewährleistet, basiert auf der Inhibition der Formation von homotypischen Zell-Aggregaten (S49.1-Zellen), die aufgrund einer spezifischen Interaktion des Fc-Teils eines gebundenen anti-Thy-1 IgG Antikörpers mit dem zellständigen FcγRezeptor II entstehen. Klone, die in diesem biologischen Test durch besondere inhibitorische Aktivität auffielen, stellten das Material dar, aus dem nun mit einem indirekten Immuntest mit Fang-Antikörper die sezemierten Antikörper weiter charakterisiert wurden. Im indirekten Immuntest wurden käufliche Antikörper verwendet, die entweder an eine Festphase (Oberfläche der Kavität einer spezielle für ELISA vertriebenen Mikrotiterplatte) gebunden vorlagen oder als markierte Antikörper das Vorhandensein der Maus-Leichtkette durch eine Farbreaktion anzeigten. Die im biologischen Test positiven, im indirekten ELISA dagegen negativen Klone wurden isoliert und zur weiteren Analyse expandiert. Um die Wahrscheinlichkeit für Monoklonalität und Stabilität zu erhöhen, wurden die isolierten Klone dem oben beschriebenen Verfahren zweimal unterworfen. Dabei gelang es uns einen Klon (2.4G2uk) zu isolieren, der auch nach längerer Kultivierung (<2 Monate) genetisch stabil ist (extrem geringer Anteil an Nicht-Produzenten; <0,01%) und den gewünschten Antikörper produziert.

Aus der Literatur ist kein vergleichbares Hybridom bekannt.

Die einzigartige Spezifität des Hybridoms 2.4G2 bereichert der Subklon 2.4G2uk durch die Eigenschaft, ausschließlich funktionell aktive Antikörper zu produzieren, d.h. Antikörper, die nur von Genen der parentalen Ratten B-Zelle kodiert werden. Folgerichtig ist die Blockade der niedrig-affinen FcγRezeptoren durch diese Antikörper auch dann noch deutlich nachweisbar, wenn die blockierende Aktivität der Antikörper des Ursprungshybridoms 2.4G2 bereits unwirksam geworden ist.

Abbildung 1 zeigt den Nachweis von Maus-κ-Ketten in Antikörpern mit Hilfe eines direkten Immuntests. Bei diesem ELISA (enzyme-linked immunosorbent assay) werden die zu untersuchenden Antikörper direkt an die Festphase (Plastikoberfläche der Kavität von ELISA-Platten) gebunden. Im vorliegenden Fall ist der Nachweis-Antikörper enzymmarkiert (Meerrettich-Peroxidase) und wird über seine spezifische Interaktion mit der Maus-κ-Kette an die Festphase gebunden. Gebundene Maus-κ-Kette-spezifische Antikörper können jetzt mit Hilfe einer von dem eingebrachten Enzym katalysierten Farbreaktion nachgewiesen werden. Die Peroxidase-katalysierte Substratumsetzung läßt man solange ablaufen, wie eine mitgeführte Negativkontrolle sich nicht verfärbt. Die Intensität der Farbreaktion wurde durch photometrische Auswertung (O.D.) mittels eines sog. 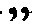 Microplate-Readers" bei einer Wellenlänge von 405 nm quantifiziert. Durch entsprechende Kontrollansätze wurde in Vorversuchen nachgewiesen, dass eingesetzten Antikörper vergleichbar gut auf der Plastikoberfläche der ELISA-Platte adsorbiert werden.

## Patentansprüche

1. Aus der Hybridomzellinie 2.4G2 (ATCC HB-197) generierte Zellinie, die keine oder zumindest weniger als 0,1% an Zellen enthält, die Misch-Antikörper produzieren, die in einem oder beiden Bindungsarmen Maus-kappa-Ketten tragen.

2. Zellinie nach Anspruch 1, dadurch gekennzeichnet, daß sie weniger als 0,05% an Misch-Antikörpern produzierenden Zellen enthält.

3. Zellinie nach Anspruch 2, dadurch gekennzeichnet, daß sie weniger als 0,01% an Misch-Antikörpern produzierenden Zellen enthält.

4. Zellinie nach Anspruch 3, dadurch gekennzeichnet, daß sie die Zellinie 2.4G2uk (DSM ACC2379) ist.

5. Verfahren zur Herstellung einer Zellinie nach einem oder mehreren der vorhergehenden Ansprüche 1-4, dadurch gekennzeichnet, daß die Zellinie 2.4G2 mutagenen Agenzien ausgesetzt und durch anschließende Selektion eine Zellinie generiert wird, die keine oder zumindest weniger als 0,1% an Zellen enthält, die Misch-Antikörper produzieren, die in einem oder beiden Bindungsarmen Maus-kappa-Ketten tragen.

6. Verwendung einer Zellinie nach einem oder mehreren der vorhergehenden Ansprüche 1-4 zur Untersuchung der Funktion von Fcγ-Rezeptoren.
